# EUROPÄISCHE PATENTSCHRIFT

(11) **EP 1 923 698 B1**
(45) Veröffentlichungstag und Bekanntmachung des Hinweises auf die Patenterteilung: **18.08.2010**
(21) Anmeldenummer: 07119174.6
(22) Anmeldetag: 24.10.2007
(51) Int. Cl.: G01N 27/419, G01N 27/407

(54) **Festelektrolyt-Gassensor**
Gas sensor
Capteur de gaz

(30) Priorität: 16.11.2006 DE 102006054183
(43) Veröffentlichungstag der Anmeldung: 21.05.2008
(73) Patentinhaber: ROBERT BOSCH GMBH, 70442 Stuttgart (DE)
(72) Erfinder: Diehl, Lothar, 70839, Gerlingen (DE); Seiler, Thomas, 70195, Stuttgart (DE)

(56) Entgegenhaltungen:
- EP-A- 0 990 895
- DE-A1- 19 622 625
- DE-A1-102005 018 436
- US-A- 5 433 830
- US-A1- 2005 284 772
- US-A1- 2006 011 476

## Beschreibung

Die Erfindung betrifft einen Gassensor nach der Gattung des Anspruchs 1.

### Stand der Technik

Elektrochemische Gassensoren in Form von Lambda-Sonden werden in großer Zahl in Abgassystemen von Verbrennungsmotoren in Kraftfahrzeugen eingesetzt, um für die Motorsteuerung Signale über die Abgaszusammensetzung bereitstellen zu können. Auf diese Weise kann der Motor so betrieben werden, dass die Abgase eine optimale Zusammensetzung für die Nachbehandlung mit im Abgassystem heute üblicherweise vorhandenen Katalysatoren aufweisen.

US 2005/0284772 A1 offenbart eine Lambda-Sprungssonde, bei der Rechteckpulse mit entgegengesetzter Polarität und gleicher Amplitude an die Elektroden angelegt werden. In der Pause zwischen den Pulsen wird entweder der Spannungsabfall oder der Strom gemessen.

Aus DE 196 22 625 A1 geht eine Vorrichtung und ein Verfahren zum Diagnostizieren einer Verschlechterung oder einer Funktionsstörung eines Sauerstoffsensors durch Anlegen einer Abfragespannung hervor.

DE 10 2005 018 436 A1 offenbart eine Vorrichtung zum Betreiben eines Sensorelements, wobei eine Elektrode in zyklisch ablaufenden Vorgängen impulsartig mit einem konstanten Pumpstrom beaufschlagt wird.

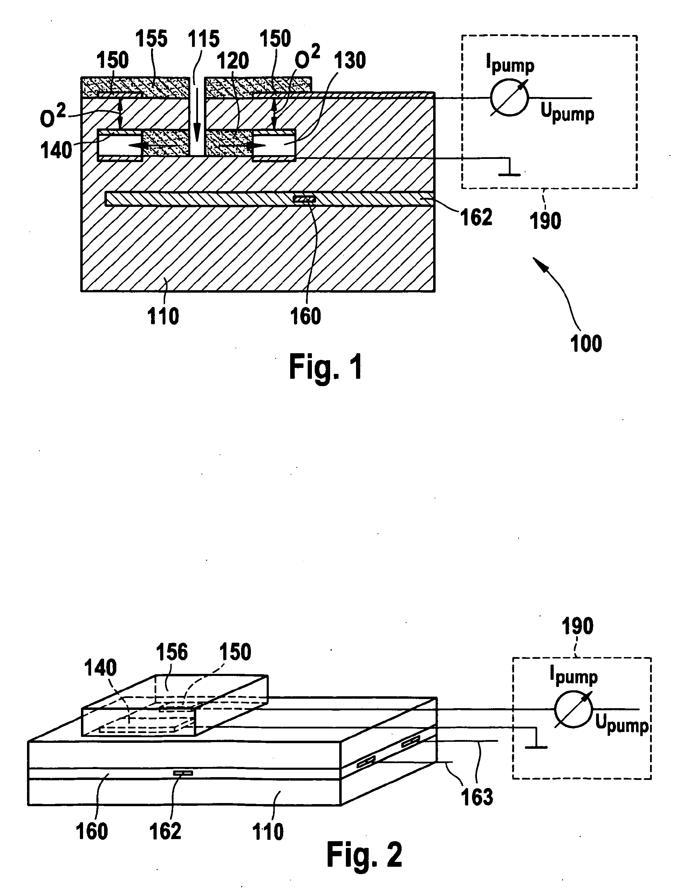

In Fig. 1 ist ein aus dem Stand der Technik bekannter gattungsgemäßer Gassensor dargestellt. Das Sensorelement 100 weist ein Gaszutrittsloch 115 auf, durch welches Abgas einströmt bzw. eindiffundiert und durch eine Diffusionsbarriere 120 in einen Messraum 130 gelangt. Eine erste Elektrode, auch als Außenelektrode oder äußere Pumpelektrode 150 bezeichnet, ist an der Außenseite des Festelektrolyten 110 und unter einer porösen Schutzschicht 155 angeordnet dem Abgas einer (nicht dargestellten) Brennkraftmaschine ausgesetzt. In dem Messraum ist eine zweite Elektrode, auch als Innenelektrode oder innere Pumpelektrode 140 bezeichnet, angeordnet.

Zwischen die innere Pumpelektrode 140 und die äußere Pumpelektrode 150 wird eine Pumpspannung Uₚᵤₘₚ angelegt, sodass ein Pumpstrom Iₚᵤₘₚ fließt. In dem Festelektrolyten 110 ist ferner eine in eine Isolationsschicht 162 eingebettete Heizung 160 angeordnet. Durch diese Heizung 160 wird das Sensorelement auf eine Temperatur erwärmt, die eine optimale Funktion des Sensorelements 100 gestattet.

Diese planare Breitband-Lambda-Sonde nach dem Grenzstromprinzip wird mit einer festen Pumpspannung Uₚᵤₘₚ beaufschlagt. Die feste Pumpspannung erzeugt bei einem mageren Abgas, d.h. bei einem Abgas mit Luftüberschuss, einen positiven Pumpstrom |ₚᵤₘₚ, der mit dem Sauerstoffgehalt des Abgases eindeutig zusammenhängt. In einem fetten Abgas, d.h. einem Abgas mit Kraftstoffüberschuss, kommt es jedoch aufgrund der Zersetzung des im Abgas enthaltenen Wassers an der inneren Pumpelektrode 140 ebenfalls zu einem positiven Pumpstrom. Die angelegte Pumpspannung Uₚᵤₘₚ liegt zwar deutlich unter der Zersetzungsspannung des Wassers, da aber Wasserstoff im Abgas existiert, wird die Wasserzersetzung energetisch möglich, denn an der Außenpumpelektrode 150 wird aus der Reaktion des Wasserstoffs mit den Sauerstoffionen Wasser erzeugt. Im Mageren ist der Pumpstrom durch die Andiffusion des Sauerstoffs zur Innenelektrode, d.h. Kathode limitiert, während die Sauerstoff-Erzeugung an der äußeren Pumpelektrode 150 nur wenig Energie erfordert. Deshalb fällt der Großteil der angelegten Pumpspannung Uₚᵤₘₚ als Konzentrationsüberspannung an der Grenzfläche zwischen der Innenelektrode (Kathode) und dem das Sensorelement bildenden Elektrolyten ab. Im Fetten ist der Pumpstrom |ₚᵤₘₚ durch die Andiffusion von Wasserstoff zur Außenelektrode (Anode) limitiert. In diesem Fall fällt der Großteil der angelegten Pumpspannung Uₚᵤₘₚ als Konzentrationsüberspannung an der Grenzfläche zwischen der Außenelektrode (Anode) und dem das Sensorelement bildenden Elektrolyten ab. Der Pumpstrom |ₚᵤₘₚ wird also bei fettem Abgas durch den Wasserstoffgehalt begrenzt. Da dieser Pumpstrom |ₚᵤₘₚ bei fettem Abgas dieselbe Richtung aufweist wie der Pumpstrom |ₚᵤₘₚ bei magerem Abgas, kann aus dem Pumpstrom |ₚᵤₘₚ nicht mehr ohne weiteres auf die Abgaszusammensetzung geschlossen werden.

### Offenbarung der Erfindung

### Vorteile der Erfindung

Der erfindungsgemäße Gassensor mit den Merkmalen des unabhängigen Anspruchs hat demgegenüber den Vorteil, dass nicht nur bestimmt werden kann, ob ein mageres Abgas (Magergas) oder ein fettes Abgas (Fettgas) vorliegt, sondern dass sowohl bei Vorliegen eines mageren Abgases als auch bei Vorliegen eines fetten Abgases auch eindeutig auf die Abgaszusammensetzung geschlossen werden kann.

Grundidee der Erfindung ist es, die Pumpspannung rechteckförmig um einen vorgebbaren Betrag zu erhöhen. In diesem Falle fällt die zusätzliche Pumpspannung fast ausschließlich an derjenigen Elektrode ab, an der die Diffusionslimitierung durch den Gas-Antransport vorliegt. Der damit verbundene zusätzliche Stromfluss rührt nur sehr wenig von einer Zunahme der Sauerstoffreduktion bzw. Wasserstoffoxidation her. Der sich dabei ergebende peakartige Anstieg mit anschließendem exponentiellem Abfall des Stromverlaufs stellt vielmehr eine Aufladekurve der elektrochemischen Doppelschicht dar. Die Vergrößerung einer die elektrochemisch aktive Fläche charakterisierenden Größe, insbesondere die geometrische Ausdehnung der Elektrodenfläche oder die Feinstruktur der einen Elektrode, insbesondere der Kathode, führt dazu, dass die Doppelschichtkapazität der Kathode erheblich größer ist als die der Anode. Wird der rechteckförmige Spannungspuls in diesem Falle bei Vorliegen eines Magergases angelegt, dann ist der hieraus sich ergebende Aufladestrom der Doppelschicht größer als bei Vorliegen eines Fettgases. Hierdurch ist eine qualitative Bestimmung möglich, ob ein Fettgas oder ein Magergas vorliegt. Der Betrag des Pumpstroms vor dem Puls bzw. nach Abklingen der Aufladekurve ermöglicht eine quantitative Bestimmung der Sauerstoff- und/oder der Wasserstoffkonzentration.

Durch die in den abhängigen Ansprüchen aufgeführten Maßnahmen sind vorteilhafte Weiterbildungen und Verbesserungen des in dem unabhängigen Anspruch angegebenen Gassensors möglich.

Gemäß Anspruch 1 ist vorgesehen, dass der Betrag der Differenz des Pumpstroms vor dem Spannungspuls und nach Ablauf einer vorgebbaren Zeitspanne nach dem Spannungspuls in der elektronischen Schaltung ausgewertet werden, um daraus auf die Zusammensetzung des Kraftstoff-Luft-Gemisches zu schließen. Wenn der Betrag der Differenz dabei größer ist als ein bestimmter vorgebbarer Schwellenwert, wird auf das Vorliegen eines Magergases geschlossen. Ist er kleiner als der Schwellenwert, wird auf das Vorliegen eines Fettgases geschlossen.

Gemäß Anspruch 1 ist auch vorgesehen, durch die elektronische Schaltung eine zwischen der ersten Elektrode und der zweiten Elektrode anliegende oszillierende Pumpspannung zu erzeugen, die um einen vorgebbaren Wert mit einer vorgebbaren Amplitude und einer vorgebbaren Frequenz im Wesentlichen rechteckförmig oszilliert.

Die Auswertung des oszillierenden Pumpstroms erfolgt in der elektronischen Schaltung, wobei aus dem oszillierenden Pumpstrom auf die Zusammensetzung des Kraftstoff-Luft-Gemisches geschlossen wird. Dies geschieht bevorzugt dadurch, dass dann, wenn die Amplitude des Pumpstroms einen vorgebbaren Schwellenwert überschreitet, auf ein Magergas geschlossen wird, und dann, wenn die Amplitude einen vorgebbaren Schwellenwert unterschreitet, auf ein Fettgas geschlossen wird.

Neben der bereits vorstehend erwähnten geometrischen Ausdehnung der Elektrodenfläche kann als eine die elektrochemisch aktive Fläche charakterisierende Größe auch die elektrochemisch aktive Ausweitung der 3-Phasenzone anhand der Variation des Stützgerüstgehalts oder die Feinstruktur verwendet werden. Die Vergrößerung erfolgt dabei um mindestens den Faktor 10.

### Kurze Beschreibung der Zeichnungen

Ausführungsbeispiele der Erfindung sind in den Zeichnungen dargestellt und in der nachfolgenden Beschreibung näher erläutert.

Es zeigen:
- Fig. 1: eine Schnittdarstellung eines aus dem Stand der Technik bekannten Gassensors;
- Fig. 2: schematisch einen von der Erfindung Gebrauch machenden Gassensor und
- Fig. 3a: der zeitliche Verlauf der Pumpspannung sowie
- Fig. 3b: der zeitliche Verlauf des sich daraus ergebenden Pumpstroms bei einem in Fig. 2 dargestellten Gassensor.

### Ausführungsformen der Erfindung

Ein aus dem Stand der Technik bekannter, in Fig. 1 dargestellter Gassensor weist ein Sensorelement 100 auf, das durch einen in Schichtbauweise ausgebildeten Festelektrolyten 110 gebildet wird. Auf der Außenseite des Sensorelements 100 ist dem Abgas ausgesetzt eine erste Elektrode, auch als äußere Pumpelektrode 150 bezeichnet, angeordnet, die durch eine offenporige Schutzschicht 155 überdeckt ist. In dem Festelektrolyten 110 ist ein Messvolumen 130 ausgebildet, in dem eine zweite Elektrode, auch als innere Pumpelektrode 140 bezeichnet, angeordnet ist. Das Abgas eines (nicht dargestellten) Verbrennungsmotors strömt durch ein Gaszutrittsloch 115 über eine Diffusionsbarriere 120 in das Messvolumen 130. Durch eine schematisch dargestellte elektronische Schaltung 190 wird zwischen der äußeren Pumpelektrode 150 und der in dem Messvolumen 130 angeordneten inneren Pumpelektrode 140 eine konstante Pumpspannung Uₚᵤₘₚ erzeugt. Hierdurch stellt sich bei einem mageren Abgas ein positiver Pumpstrom |ₚᵤₘₚ ein, der dazu führt, dass Sauerstoffionen O²⁻ von dem Messvolumen 130 ins Äußere des Sensorelements, in das Abgas gepumpt werden. Bei einer fetten Abgaszusammensetzung, d.h. bei einem Kraftstoffüberschuss des Abgases kommt es jedoch aufgrund der Zersetzung des im Abgas enthaltenen Wassers ebenfalls zu einem positiven Pumpstrom. Die angelegte Pumpspannung liegt dabei zwar deutlich unter der Zersetzungsspannung des Wassers. Da aber Wasserstoff im Abgas existiert, wird die Wasserzersetzung energetisch möglich, denn an der äußeren Pumpelektrode 150 wird aus H₂ und O₂ Wasser erzeugt. Der Strom wird also bei einer fetten Abgaszusammensetzung durch den Wasserstoffgehalt an der Außenelektrode begrenzt. Da der Pumpstrom Iₚᵤₘₚ bei fetter Abgaszusammensetzung dieselbe Richtung aufweist wie der Pumpstrom Iₚᵤₘₚ bei magerer Abgaszusammensetzung kann nicht ohne weiteres aus dem Pumpstrom auf die Abgaszusammensetzung geschlossen werden.

Um nunmehr auf die Abgaszusammensetzung schließen zu können, wird bei einem Gassensor die geometrische Ausdehnung einer der beiden Elektroden, bevorzugt der Kathode 140 im Vergleich zur Anode 150 signifikant größer ausgebildet oder die Feinstruktur dieser Elektrode entsprechend verändert. Dies kann beispielsweise dadurch geschehen, dass die elektrochemisch aktive Oberfläche der Kathode 140 durch Vergrößerung der geometrischen Ausdehnung, aber auch durch Ausweitung der sogenannten 3-Phasenzone anhand der Variation des Stützgerüstgehalts, also des Verhältnisses von Platin zu Zirkonoxid, um mindestens den Faktor 10 vergrößert gegenüber der elektrochemisch aktiven Oberfläche der Anode 150 oder umgekehrt.

Beide Elektroden 140, 150 sind dabei von einer gemeinsamen Schutzschicht 156 oder auch von jeweils einer getrennten Schutzschicht überdeckt (nicht dargestellt), sie sind vorzugsweise auf der Außenseite des das Sensorelement bildenden Festelektrolyten 110 angeordnet, der durch eine Heizung 162, die in einer Heizungsschutzschicht 160 eingebettet ist und entsprechende Zuleitungen 163 aufweist, auf einen vorgebbaren, für den Betrieb des Gassensors erforderliche Temperatur beheizbar ist.

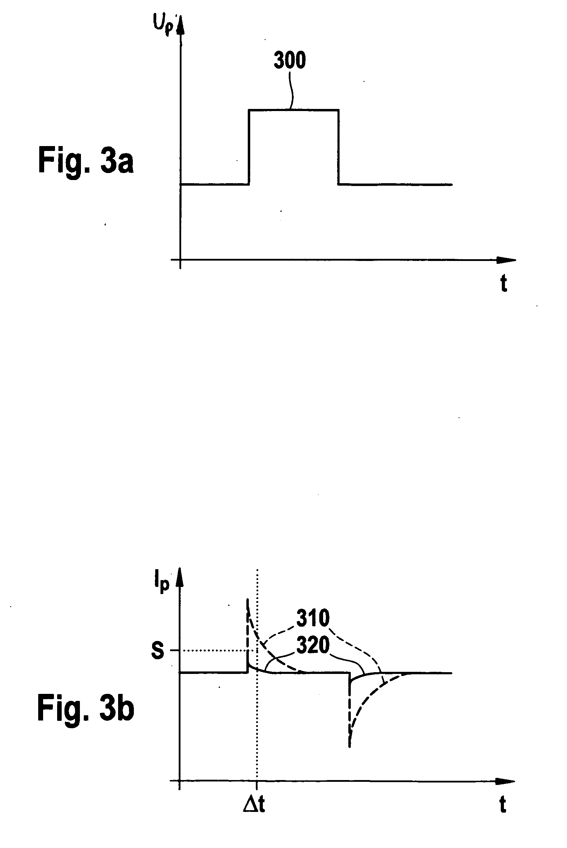

Als nicht erfindungsgemäßes Ausführungsbeispiel wird die Pumpspannung Uₚ wird nun rechteckförmig um einen festen Betrag, vorzugsweise um 200 mV erhöht, wie es in Fig. 3a schematisch dargestellt ist. In diesem Falle fällt die zusätzliche Pumpspannung fast ausschließlich an derjenigen Elektrode ab, an der die Diffusionslimitierung durch den eingangs beschriebenen Gas-Antransport vorliegt. Der damit verbundene zusätzliche Stromfluss rührt nur sehr wenig von einer Zunahme der Sauerstoffreduktion beziehungsweise der Wasserstoffoxidation her. Der Pumpstrom Iₚ erfährt einen peakartigen Anstieg mit anschließendem exponentiellem Abfall. Dieser Stromverlauf stellt im Wesentlichen die Aufladekurve der elektrochemischen Doppelschicht dar.

Aufgrund der größeren geometrischen Ausdehnung der Kathode 140 im Vergleich zur Anode 150 ist die Doppelschichtkapazität der Kathode 140 erheblich größer als die der Anode. Wenn in diesem Falle daher der rechteckförmige Puls 300 bei Vorliegen eines Magergases angelegt wird, dann ist der hieraus resultierende Aufladestrom der Doppelschicht, in Fig. 3b mit 310 bezeichnet, größer als der Aufladestrom 320 bei Vorliegen eines Fettgases. Durch diesen Unterschied des Aufladestroms kann qualitativ bestimmt werden, ob ein Fett- oder Magergas vorliegt. Durch Bestimmung des Betrags des Pumpstroms vor dem Puls beziehungsweise nach Abklingen der Aufladekurve kann auch eine quantitative Konzentration entweder von Sauerstoff oder Wasserstoff bestimmt werden. Ausgewertet wird hierbei der Betrag der Differenz des Pumpstroms vor dem Sprung und des Pumpstroms nach Ablauf einer definierten Zeitspanne nach dem Sprung, vorzugsweise nach Ablauf einer Zeitspanne Δt von vorzugsweise zwischen 1 bis 10 msec. Ist dieser Betrag größer als ein bestimmter Schwellenwert S, so wird darauf geschlossen, dass ein Magergas vorliegt, ist er kleiner als der Schwellenwert S, so wird darauf geschlossen, dass ein Fettgas vorliegt.

In Fig. 3b ist diese Auswertung schematisch anhand des positiven Peaks (in Richtung des zunehmenden Pumpstroms) entsprechend dargestellt.

Der Potenzialsprung kann entweder periodisch oder nach jedem Nulldurchgang des Pumpstroms durchgeführt werden. Dabei ist ein unmittelbar anschließender Potenzialrücksprung nicht erforderlich. Beträgt beispielsweise die Pumpspannung zum Zeitpunkt einer Messung, also nach dem Nulldurchgang des Pumpstroms 500 mV, dann erfolgt ein Sprung auf 700 mV. Bei dieser Spannung wird der Sensor dann weiterbetrieben. Beträgt die Pumpspannung zum Zeitpunkt der Messnotwendigkeit 700 mV, dann erfolgt ein Sprung auf 500 mV.

Erfindungsgemäß ist vorgesehen, die Pumpspannung Uₚ oszillierend um einen vorgegebenen Wert, beispielsweise um 600 mV mit einer vorgebbaren Amplitude von beispielsweise 100 mV und einer vorgebbaren Frequenz, die vorzugsweise zwischen 100 Hz und 1 KHz liegt, auszuführen. Die resultierende Amplitude des in diesem Falle ebenfalls oszillierenden Pumpstroms Iₚ liefert dann die qualitative Information über die Abgaszusammensetzung. Bei Überschreiten eines vorgebbaren Schwellenwertes liegt ein Magergas vor, bei Unterschreiten ein Fettgas. Der Mittelwert des Pumpstroms liefert die quantitative Information über die Gaskonzentration.

In beiden Fällen kann auch vorgesehen sein, die Kathode 140 mit dem Masseanschluss der Heizung elektrisch zu verbinden. Bei dieser Ausführungsform muss die Frequenz, mit der die Pumpspannung Uₚ oszilliert, größer sein als die Frequenz der Heizertaktung, um Interferenzen zu vermeiden. Um derartige Interferenzen zu vermeiden, kann rein prinzipiell auch vorgesehen sein, die Heizung während einer definierten Messphase auszuschalten.

## Patentansprüche

1. Gassensor, insbesondere Lambda-Sonde, für die Bestimmung der Sauerstoffkonzentration im Abgas einer mit einem Kraftstoff-Luft-Gemisch betriebenen Brennkraftmaschine umfassend eine in oder an einem Sensorelement (110) angeordnete Pumpzelle mit einer ersten Elektrode (150) und mit einer zweiten Elektrode (140), die von dem Abgas durch wenigstens eine Schicht (155; 156) getrennt sind, und mit einer elektronischen Schaltung (190) zur Erzeugung einer zwischen der ersten Elektrode (150) und der zweiten Elektrode (140) anliegenden Spannung (Uₚᵤₘₚ) und zur Messung und Auswertung eines sich dabei einstellenden Pumpstroms (Iₚᵤₘₚ), um daraus auf die Zusammensetzung des Kraftstoff-Luft-Gemisches zu schließen, wobei eine die elektrochemisch aktive Fläche entweder der ersten Elektrode (150) oder der zweiten Elektrode (140) charakterisierende Größe in Vergleich zu der jeweils anderen Elektrode vergrößert ist und wobei durch die elektronische Schaltung eine zwischen der ersten Elektrode (150) und der zweiten Elektrode (140) anliegende oszillierende Pump-Spannung (Up) erzeugt wird, die um einen vorgebbaren Wert mit einer vorgebbaren Amplitude und einer vorgebbaren Frequenz im Wesentlichen rechteckförmig oszilliert, und wobei die aufgrund der oszillierenden Pumpspannung resultierende Amplitude des oszillierenden Pumpstroms in der elektronischen Schaltung (190) ausgewertet wird, um daraus auf die Zusammensetzung des Kraftstoff-Luft-Gemisches zu schließen, **dadurch gekennzeichnet, dass** dann, wenn die Amplitude des oszillierenden Pumpstroms eine vorgebbare Schwelle überschreitet, in der elektronischen Schaltung (190) ein Signal für das Vorliegen eines Magergases erzeugt wird und dann, wenn die Amplitude des oszillierenden Pumpstroms den Schwellenwert unterschreitet, in der elektronischen Schaltung (190) ein Signal für das Vorliegen eines Fettgases erzeugt wird.

2. Gassensor nach Anspruch 1, **dadurch gekennzeichnet, dass** eine die elektrochemisch aktive Fläche entweder der ersten Elektrode (150) oder der zweiten Elektrode (140) charakterisierende Größe eine, der folgenden Größen ist:
- die Ausdehnung der Elektrodenfläche,
- die Ausweitung der 3-Phasenzone anhand einer Variation des Stützgerüstgehalts,
- die Feinstruktur der Elektrode.

3. Gassensor nach Anspruch 2, **dadurch gekennzeichnet, dass** die die elektrochemisch aktive Fläche entweder der ersten Elektrode (150) oder der zweiten Elektrode (140) charakterisierende Größe um wenigstens den Faktor 10 gegenüber der Fläche der jeweils anderen Elektrode vergrößert ist.

## Claims

1. Gas sensor, in particular lambda probe, for determining the oxygen concentration in the exhaust gas of an internal combustion engine operated with a fuel-air mixture, comprising a pump cell which is arranged in or on a sensor element (110) and has a first electrode (150) and a second electrode (140), which are separated from the exhaust gas by means of at least one layer (155; 156), and an electronic circuit (190) for generating a voltage (Uₚᵤₘₚ) applied between the first electrode (150) and the second electrode (140) and for measuring and evaluating a pump current (Iₚᵤₘₚ) which is established in the process in order to infer the composition of the fuel-air mixture therefrom, a variable which characterizes the electrochemically active surface of either the first electrode (150) or the second electrode (140) being increased in comparison with the respective other electrode, and the electronic circuit generating an oscillating pump voltage (Up) which is applied between the first electrode (150) and the second electrode (140) and oscillates in essentially square-wave form by a predefinable value with a predefinable amplitude and at a predefinable frequency, and the amplitude of the oscillating pump current, which results from the oscillating pump voltage, being evaluated in the electronic circuit (190) in order to infer the composition of the fuel-air mixture therefrom, **characterized in that**, when the amplitude of the oscillating pump current exceeds a predefinable threshold, a signal for the presence of a lean gas is generated in the electronic circuit (190) and, when the amplitude of the oscillating pump current undershoots the threshold value, a signal for the presence of a fat gas is generated in the electronic circuit (190).

2. Gas sensor according to Claim 1, **characterized in that** a variable which characterizes the electrochemically active surface of either the first electrode (150) or the second electrode (140) is one of the following variables:
- the extent of the electrode surface,
- the expansion of the 3-phase zone on the basis of a variation in the supporting structure content,
- the fine structure of the electrode.

3. Gas sensor according to Claim 2, **characterized in that** the variable which characterizes the electrochemically active surface of either the first electrode (150) or the second electrode (140) is increased by at least a factor of 10 in comparison with the surface of the respective other electrode.

## Revendications

1. Détecteur de gaz, en particulier sonde lambda, destiné à déterminer la concentration en oxygène dans les gaz d'échappement d'un moteur à combustion interne alimenté en un mélange d'air et de carburant et comprenant
une cellule de pompage disposée dans ou sur un élément de détection (110) et dotée d'une première électrode (150) et d'une deuxième électrode (140) séparées des gaz d'échappement par au moins une couche (155; 156),
un circuit électronique (190) qui forme une tension (Uₚᵤₘₚ) appliquée entre la première électrode (150) et la deuxième électrode (140) et qui mesure et évalue le courant de pompage (Iₚᵤₘₚ) qui s'établit à cette occasion pour en tirer des conclusions sur la composition du mélange d'air et de carburant,
une grandeur qui caractérise la surface électrochimiquement active de la première électrode (150) ou de la deuxième électrode (140) étant augmentée par rapport à l'autre électrode,
le circuit électronique formant une tension oscillante de pompage (Up) appliquée entre la première électrode (150) et la deuxième électrode (140) qui oscille de manière essentiellement rectangulaire autour d'une valeur prédéterminée, avec une amplitude prédéterminée et à une fréquence prédéterminée,
l'amplitude du courant oscillant de pompage qui résulte de l'oscillation de la tension de pompage étant évaluée dans le circuit électronique (190) pour ainsi tirer des conclusions sur la composition du mélange d'air et de carburant,
**caractérisé en ce que**
lorsque l'amplitude du courant oscillant de pompage dépasse un seuil prédéterminé, un signal de présence d'un gaz pauvre est formé dans le circuit électronique (190) et
**en ce que** lorsque l'amplitude du courant oscillant de pompage n'atteint pas la valeur de seuil, un signal de présence d'un gaz riche est formé dans le circuit électronique (190).

2. Détecteur de gaz selon la revendication 1, **caractérisé en ce que** la grandeur qui caractérise la surface électrochimiquement active de la première électrode (150) ou de la deuxième électrode (140) est une des grandeurs suivantes :
- l'extension de la surface de l'électrode,
- un élargissement de la zone à 3 phases à l'aide d'une variation de la teneur en ossature de soutien ou
- la structure fine de l'électrode.

3. Détecteur de gaz selon la revendication 2, **caractérisé en ce que** la grandeur qui caractérise la surface électrochimiquement active de la première électrode (150) ou de la deuxième électrode (140) est augmentée d'un facteur d'au moins 10 par rapport à la surface de l'autre électrode.
